**Europäisches Patentamt**

**European Patent Office**

**Office européen des brevets**

(19)

(11) Veröffentlichungsnummer: **0 158 630**
**B1**

(12) # EUROPÄISCHE PATENTSCHRIFT

(45) Veröffentlichungstag der Patentschrift:
**15.11.89**

(21) Anmeldenummer: **85890092.1**

(22) Anmeldetag: **04.04.85**

(51) Int. Cl.⁴: **A 61 N 5/00**, A 61 M 37/00

(54) **Vorrichtung zum gefahrlosen Einbringen radioaktiver Strahlenquellen in interstitiell eingebrachte Bestrahlungsvorrichtungen.**

(30) Priorität: **10.04.84 AT 1203/84**

(43) Veröffentlichungstag der Anmeldung:
**16.10.85 Patentblatt 85/42**

(45) Bekanntmachung des Hinweises auf die Patenterteilung:
**15.11.89 Patentblatt 89/46**

(84) Benannte Vertragsstaaten:
**AT CH DE FR GB LI**

(56) Entgegenhaltungen:
**EP-A-0 152 124**
**DE-A-1 945 015**
**DE-B-1 271 272**
**DE-B-2 009 356**
**DE-C-522 404**
**GB-A-1 027 078**

**BRITISH JOURNAL OF RADIOLOGY, Band 48, Nr 568, April 1975, Seiten 295-298, British Institute of Radiology, London, GB; J.L. HAYBITTLE et al.: "A simple after-loading technique for the treatment of cancer of the cervix"**

(73) Patentinhaber: **Österreichisches Forschungszentrum Seibersdorf Ges.m.b.H., Kramergasse 1, A-1010 Wien (AT)**

(72) Erfinder: **Schöberl, Erich, Dipl.- Ing., Hadikgasse 128/3/40, A-1140 Wien (AT)**
Erfinder: **Prager, Leopold, Hetzendorferstrasse 96/3/1, A-1120 Wien (AT)**
Erfinder: **Miksche, Wolfgang, Fischergasse 6, A-2333 Leopoldsdorf (AT)**
Erfinder: **Neubauer, Josef, Bernoullistrasse 4/21/6/14, A-1220 Wien (AT)**

EP 0 158 630 B1

## Beschreibung

Die Erfindung bezieht sich auf eine medizinisch-therapeutische Vorrichtung zum gefahrlosen Einbringen von radioaktiven Strahlenquellen in in den menschlichen Körper eingebettete Bestrahlungsvorrichtungen, vorzugsweise in interstitiell eingebrachte, hohle Bestrahlungsnadeln, insbesondere zur low-dose-rate-Bestrahlung, wobei die Vorrichtung aus einem oder mehreren identisch aufgebauten Modulen, bestehend aus je einer strahlenschutzmäßig abgeschirmten Belade- und bzw. oder Lagerstation für die jeweilige Strahlenquelle, je einer Beförderungseinrichtung mit einer in einer Hülle (Transportschlauch) geführten flexiblen Schubstange (Transportseil) und je einer von der Beförderungseinrichtung abkoppelbaren Bestrahlungsvorrichtung.

Die medizinische Behandlung von Geschwulsten kann in verschiedener Form erfolgen. Je nach Stadium, Lage und Art der Tumors werden neben anderen auch strahlentherapeutische Mittel angewendet. Eine Variante dabei ist die Verwendung von radioaktiven Stoffen, die - an den Tumor herangebracht - ihn auf Grund der Strahlung beseitigen. Ein Problem ist dabei die optimale Positionierung der Strahlenquellen, damit möglichst wenig gesundes Gewebe zerstört oder geschädigt wird. Ist der Tumor über eine Körperöffnung zugänglich (z . B. Uterus ), so wird ein entsprechend ausgebildeter Applikator eingesetzt, der nach genauer Einjustierung fernbedient mit einer oder mehreren Strahlenquellen beladen werden kann (intrakavitäre Therapie mit Afterloading-Technik). Wenn die Geschwulst nicht über eine Körperöffnung zugänglich ist (z. B. Brustkrebs), besteht die Möglichkeit, den Tumor mit Hohlnadeln zu spicken, wobei sich in den Hohlnadeln Strahlenquellen befinden (interstitielle Therapie).

Allgemein gesehen sollen bei der Verwendung von radioaktiven Strahlenquellen diese ohne Strahlenrisiko für das Krankenhauspersonal an den mittels Bestrahlung zu bekämpfenden Krankheitsherd (z. B. Uterus-Krebsgeschwüren) im Patienten herangebracht werden. Üblicherweise wird hiezu eine Bestrahlungsvorrichtung in den Patienten eingebracht, die genaue Bestrahlungsgeometrie einjustiert und anschließend eine oder mehrere radioaktive Strahlenquellen in die Bestrahlungseinrichtung eingebracht. Üblicherweise werden zu diesem Zweck entweder pneumatische Verfahren angewendet oder Strahlenquellen mittels Stahlseile in Schlauchführungen bewegt, wobei entweder die Strahlenquelle an das Seil gekuppelt ist oder ein Strahlerhalter, in dem sich eine oder mehrere Strahlenquellen befinden, mit dem Seil verkuppelt ist. Auf diese Weise können entweder pneumatisch oder durch die Bewegung des Stahlseiles, durch geeignete Fernbedienung, Strahlenquellen in die Bestrahlungsposition im Patienten befördert werden. Diese Technik wird mit Nachladeverfahren oder afterloading bezeichnet. Bei diesen Vorrichtungen sind Strahlenquellen mit relativ großer Dimension (Ø 2 - 5 mm) erforderlich; daher weisen die in den Patienten einzubringende Bestrahlungsvorrichtungen relativ große Durchmesser auf. Derartige Vorrichtungen können nur intrakavitär in den Patienten eingebracht werden, also nur in entsprechende Körperöffnungen (z .B. Uteruskrebs) eingesetzt werden. Ein Gerät dieser Art ist z. B. aus der DE-OS-2 009 356 bekannt. Dieser bekannte Projektor ist im wesentlichen dadurch gekennzeichnet, daß er von einem auf einem fahrbaren Gestell ruhendem Gehäuse gebildet wird und mit einem Abschirmblock für die radioaktiven Quellen und einer elektromechanischen Transportvorrichtung für diese Strahlenquellen besteht sowie mit einer Schalttafel versehen ist. Strahlenquellenträger dienen dazu, die Strahlenquellen aufzunehmen und in die Nachbarschaft oder in das zu behandelnde krebsartig veränderte Gewebe zu bringen. Eine Einrichtung für eine rasche Verbindung der Strahlenquellenträger mit einem Antriebsseil ist vorgesehen, welche letzteres selbst wiederum durch eine elektromechanische Transportvorrichtung angetrieben wird. Äußere biegsame Rohre nehmen je einen Strahlenquellenträger auf und sind über Anschlußstecker mit biegsamen Ejektionshüllen des Projektors fest verbunden. Diese Ejektionshüllen können aus dem Gehäuse ausgefahren bzw. herausgezogen werden und dienen als Leitrohre für die Strahlenquellenträger. Das Ein- und Ausfahren der Strahlenquellenträger ist jedoch aufwendig und zeitraubend.

Aus medizinischen Gründen sind verschiedene Formen von Strahlenquellen mit unterschiedlichen Aktivitäten erforderlich. Darüberhinaus werden verschiedene Therapietechniken angewandt, bei denen die derzeit angebotenen Afterloading-Vorrichtungen verwendet werden können. So ist es zum Beispiel bei Brustkrebs erforderlich, ein oder mehrere Strahlenquellen interstitiell in das Brustgewebe der Patientin in eine vorher festzulegende Geometrie einzubringen und nach der Strahlenbehandlung wieder zu entfernen. Für diese Art der Therapie finden sehr dünne längliche, insbesondere drahtförmige Strahlenquellen mit verschiedenen Längen Verwendung. Ferner ist bei der Therapietechnik mit den herkömmlichen Afterloading-Vorrichtungen der Patient in seiner Bewegungsfreiheit sehr stark eingeschränkt, da bei diesen die Strahlenquellen mit den Beförderungsvorrichtungen in Verbindung bleiben, was bei einer für die interstitielle Therapie typischen Bestrahlungsdauer von ca. drei Tagen eine erhebliche physische und/oder psychische Belastung des Patienten darstellt, bzw. die Anwendung ausschließt. Bei Anwendung der interstitiellen Strahlentherapie werden daher derzeit üblicherweise die bereits jeweils mit der Strahlenquelle beladenen Bestrahlungsvorrichtungen in den Patienten eingebracht und entsprechend der erforderlichen Bestrahlungsgeometrie einjustiert. Eine andere Vorgangsweise besteht darin, daß zunächst die leere Bestrahlungsvorrichtung (Hohlnadel) eingebracht wird und anschließend diese von Hand aus mit der Strahlenquelle beladen wird. Beide Vorgangsweisen bedingen eine erhöhte, bei wiederholter Durchführung unzulässige Strahlenbelastung des behandelnden Arztes.

Die erfindungsgemäße Vorrichtung hat zum Ziel, die oben angeführten Belastungen zu vermeiden. Erfindungsgemäß wird diese Belastung bei einer Vorrichtung der eingangs genannten Art dadurch vermieden, daß keine Verbindung zwischen der Strahlenquelle und der flexiblen Schubstange der Beförderungseinrichtung vorgesehen ist, so daß die Strahlenquelle in der Bestrahlungsposition verbleibt, wenn nach Erreichen der Bestrahlungsposition die flexible Schubstange in die Ausgangslage zurückgeführt wird, oder daß zwischen der Strahlenquelle und der flexiblen Schubstange der Beförderungseinrichtung eine in der Bestrahlungsposition die Verbindung zwischen Schubstange und Strahlenquelle freigebende Kupplung vorgesehen ist. Es können somit die mit der Beförderungseinrichtung nicht verbundenen, d. h. nur vorwärtsbewegten, bzw. lösbar verbundenen Strahlenquellen fernbedient in die Bestrahlungsvorrichtungen eingebracht werden, dort freigegeben und abgelegt werden. Dadurch kann der Arzt ohne Strahlenbelastung und damit in Ruhe und ohne Zeitdruck die Bestrahlungsvorrichtung(en), normalerweise speziell ausgebildete Hohlnadeln, beim Patienten einsetzen. Nach dem Ankuppeln der Beförderungseinrichtung und deren Betätigung können Strahlenquellen in die Hohlnadeln eingebracht und dort so lange wie notwendig belassen werden, wobei der Patient weitgehende Bewegungsfreiheit behält, da die Beförderungsvorrichtung(en) nach Einbringung der Strahlenquellen wieder abgekuppelt werden kann (können). Wenn gewünscht, können die Strahlenquellen wieder herausgeholt bzw. durch andere ersetzt werden, ohne die Bestrahlungsvorrichtung aus dem Körper entfernen zu müssen.

Bevorzugte Ausführungsformen der Erfindung sind in der folgenden Beschreibung, der Zeichnung und den Unteransprüchen enthalten.

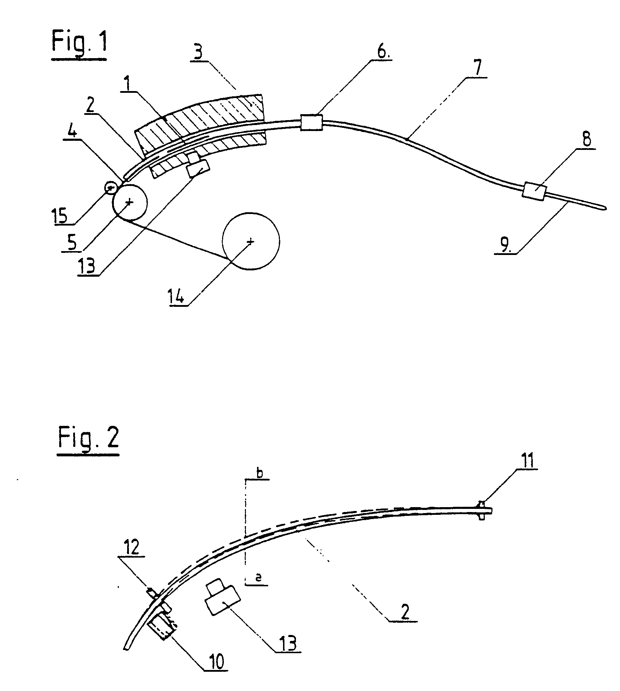

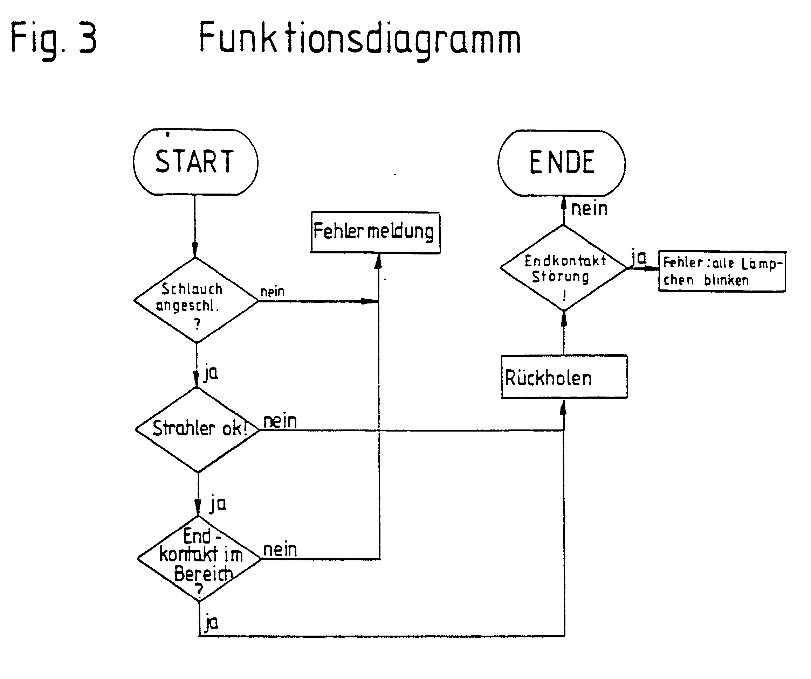

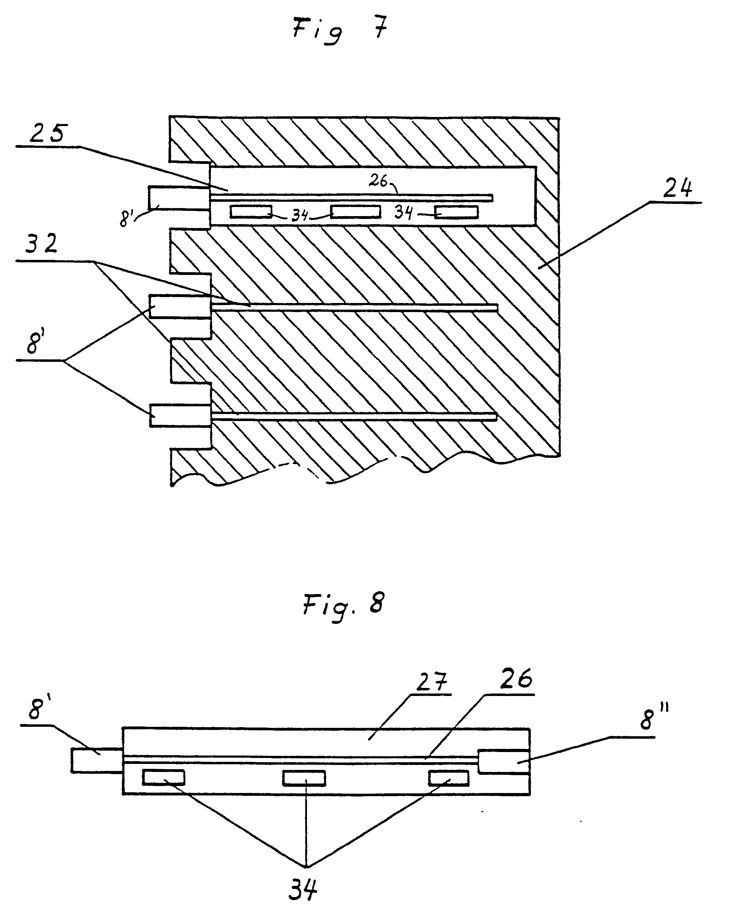

Im folgenden wird die Erfindung in nicht einschränkender Weise an Hand von Ausführungsbeispielen beschrieben. Fig. 1 zeigt schematisch eine erfindungsgemäße Vorrichtung, Fig. 2 eine Detailansicht von Fig. 1, Fig. 3 ein Funktionsdiagramm, Fig. 4a, 4b, 5a, 5b, 6a und 6b verschiedene Kupplungsvorrichtungen, Fig. 7 schematisch ein Detail eines Lagerbehälters und Fig. 8 schematisch einen mobilen Meßkanal.

Fig. 1 zeigt in schematischer Darstellung den Aufbau eines Moduls. Pro Modul wird eine Strahlenquelle 1 befördert. Sie wird in Anwendungsfall aus der Belade- und Lagerstation, die sich in einem gekrümmten Rohr 2 innerhalb einer Strahlenabschirmung 3 befindet, mit Hilfe einer flexiblen Schubstange 4 (z. B. eines plastikummantelten Stahlseils) und eines Antriebmotors (z. B. eines Schrittmotors) über die Kupplung 6 in einen Verbindungsschlauch 7 und über die Kupplung 8 in die Bestrahlungsposition der interstitiell positionierten Bestrahlungsvorrichtung 9 (Hohlnadel transportiert. Nach Erreichen der Bestrahlungsposition (Endlage) wird die flexible Schubstange wieder in die Ausgangslage zurückgespult, wobei die Strahlenquelle in der Bestrahlungsposition verbleibt. Das Schubseil wird auf einer z. B. mittels Federkraft vorgespannter Wickelspule, je nach Bewegung der von einem Schrittmotor angetriebenen Transportrolle 5, auf- bzw. abgewickelt. Die Reibung des Schubseils auf der Transportrolle 5 ist mit Hilfe der Anpreßrolle 15 so eingestellt, daß der Seiltransport nur durch den Schrittmotor bestimmt ist, und die Spule 14 nur zum selbständigen Auf- bzw. Abwickeln dient.

Wie für strahlentherapeutische Vorrichtungen allgemein erforderlich, sind mehrfach Überwachungsvorrichtungen vorgesehen, um sicherzustellen, daß die jeweilige Strahlenquelle tatsächlich bis zur Endlage befördert wurde:

1a) Mechanische Kontrolle für das Erreichen der Bestrahlungsposition

Wie aus Fig. 1 und 2 ersichtlich wird durch Erreichen der Endlage der Strahlenquelle 1 durch das Transportseil 4 innerhalb der Beförderungsstrecke ein mechanischer Druck aufgebaut, der zu einer zusätzlichen Verkrümmung des Rohres 2 führt (a...Ausgangslage, b...Erreichung der Bestrahlungsposition). Hiezu ist das Führungsrohr einseitig, z. B. in der Halterung 11, fixiert und in einer zweiten Halterung, z. B. 12, verschiebbar gelagert. Diese zusätzliche Verkrümmung des Rohres 2 wird zur Detektierung der erreichten Bestrahlungsposition verwendet. In Fig. 2 ist gezeigt, daß dies beispielsweise durch einen auf der Rohr 2 fixierten Schalter 10 erfolgt, welcher durch die Rohrbewegung mittels der Halterung 12 geschaltet wird. Durch diesen Schalter wird die Drehrichtung des Antriebsmotors geändert und das Seil 4 automatisch wieder in die Ausgangslage zurückbefördert.

1b) Alternativ zu 1a) kann das Erreichen der Bestrahlungsposition auch elektronisch, durch Überwachung der Stromaufnahme des Schrittmotors, erfolgen. In dieser Variante ist das Rohr 2 fix montiert, z. B. zwischen den Halterungen 11 und 12. Durch Erreichen der Bestrahlungsposition ändert sich der mechanische Transportwiderstand des Schubseils und damit verbunden die Stromaufnahme des Transportmotors, vorzugsweise des Schrittmotors. Eine entsprechende elektronische Vorrichtung bewirkt die Umschaltung der Motordrehrichtung.

Zusätzlich zu diesem Überwachen des Erreichens der Bestrahlungsposition ist als weitere Sicherung vorgesehen, daß die Strahlenquelle 1 nur innerhalb eines vorherbestimmten Transportwegbereichs in die Bestrahlungsposition gebracht werden darf, da die Überwachung gemäß 1a) bzw. 1b) dann unzureichend ist, wenn der Transportschlauch 7 entweder nicht angeschlossen, beschädigt oder blockiert (z. B. durch zu starke Krümmung) ist.

Da die Überwachungsvorrichtungen 1a) oder 1b) auch bei einem erhöhten mechanischen Widerstand der Transportführung z. B. durch einen beschädigten oder in zu engem Radius gelegten Verbindungsschlauch anspricht, wird elektronisch, z. B. durch Zählen der Schritte des Schrittmotors, die Beförderungsdistanz derart überwacht, daß ein Umschalten der Drehrichtung des Schrittmotors vor Erreichen einer vorwählbaren Distanz zu einer Störanzeige, beispielsweise am Steuerpult, führt. Wird andererseits bis zu einer vorgebbaren maximalen Ausfahrdistanz das Umschalten der Drehrichtung des Schrittmotors nicht ausgelöst, so wird ebenfalls z. B. über Schrittzählung eine Störung angezeigt und die Seilbewegung gestoppt. Dadurch wird ein unkontrolliertes Abrollen des Stahlseiles, beispielsweise durch eine irrtümlich nicht angekuppelte Bestrahlungsvorrichtung bedingt, vermieden.

Eine weitere Überwachung besteht darin, daß das Anschließen der Transportschlauche 7 durch geeignete Vorrichtungen erfolgt.

Eine weitere Überwachung der erfindungsgemäßen Vorrichtung besteht darin, daß beim Einleiten des Ausfahrvorganges mit Hilfe eines, z. B. in die Abschirmung 3 eingebauten, Strahlendetektors 13 geprüft wird, ob sich in der Belade- und Lagerposition eine Strahlenquelle befindet. Befindet sich in dieser keine Strahlenquelle, wird der Ausfahrvorgang nicht durchgeführt und/oder eine diesbezügliche Störmeldung ausgelöst.

Das Zusammenwirken dieser Überwachungsvorrichtungen ist in Fig. 3 in Form eines Flußdiagramms zusammengestellt.

Vorteilhafterweise werden Strahlenquellen mit einer inaktiven Umhüllung verwendet. Alternativ können auch solche Strahlenquellen verwendet werden, die aus einem mit einem anderen Material ummantelten Kern bestehen, wobei nach der gemeinsamen Aktivierung die Ummantelung, z. B. Platin, durch genügend langes Abklingen hinreichend inaktiv wird.

Die Dimensionierung der Strahlenabschirmung 3 erfolgt gemäß den einschlägigen Strahlenschutzbestimmungen. Als Abschirmungsmaterial finden vorzugsweise Blei, Schwermetalle, vorzugsweise Wolfram und/oder abgereichertes Uran Verwendung.

In den Fig. 4a, 4b, 5a, 5b, 6a bzw. 6b werden Ausführungsformen für lösbare Kupplungseinrichtungen gezeigt, bei denen das Transportseil 4 und die Strahlenquelle 1 lösbar miteinander verbunden sind, nachdem in Fig. 1 eine Anordnung besprochen wurde, bei der zwischen dem Transportseil 4 und der Strahlenquelle keine Verbindung vorhanden war und die Strahlenquelle 1 nur vorwärtsgestoßen wurde. Gemäß Fig. 4a ist die Strahlenquelle 1 von einer Umhüllung 20 umgeben und am Ende des Transportseiles 4 ist ein Klemmkörper 21 vorgesehen, der unter Anwendung eines gewissen Kraftaufwandes in das vorragende Ende der elastischen Umhüllung 20 einführbar ist und von diesem elastisch umschlossen und festgehalten wird, so daß eine lösbare Kupplung gebildet ist. Der Klemmkörper 21 verjüngt sich nach vorne, z. B. kegelig, um gut in die Umhüllung 20 einführbar zu sein, die ihrerseits im Transportschlauch 2 bzw. 7 bzw. in der Bestrahlungsvorrichtung 9 oder in den Kanälen 26, 32 (Fig. 7 und 8) leicht verschiebbar ist.

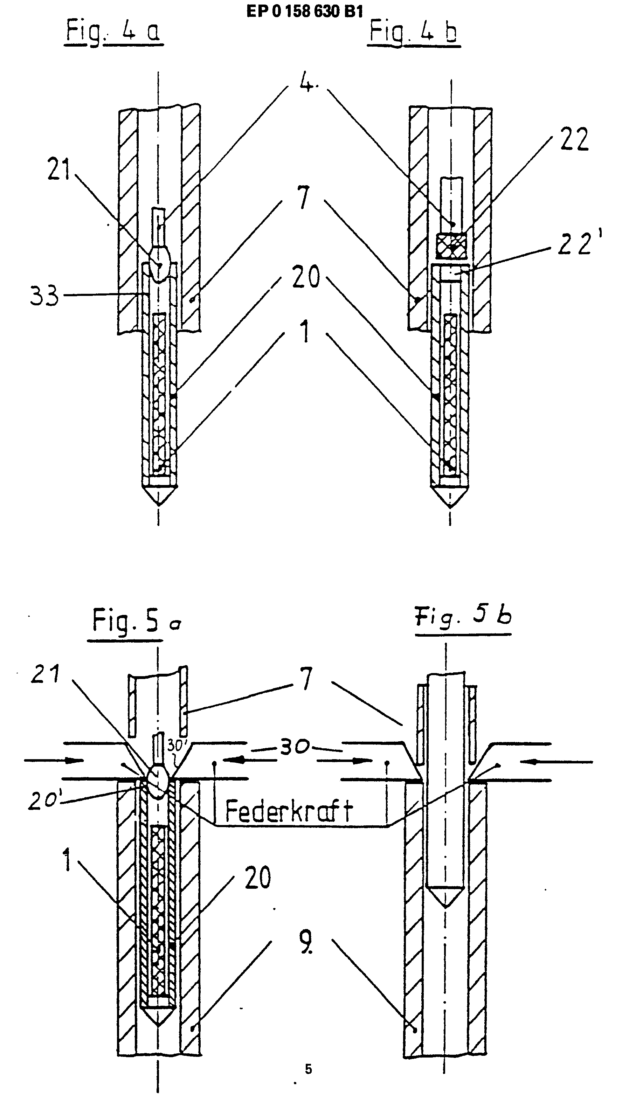

Fig. 4b zeigt einen vom Ende der Umhüllung 20 aufgenommenen Magneten bzw. magnetisierbaren Körper 22', der mit einem am Ende des Transportseiles 4 angeordneten magnetisierbaren Körper bzw. Magneten 22 zusammenwirkt und ebenfalls eine lösbare Verbindung zwischen Transportseil 4 und Strahlenquelle 1 ergibt.

Fig. 5a bzw. 5b zeigen schematisch eine Anordnung zum Entkuppeln bzw. zum Abtrennen der Umhüllung 20 vom Transportseil 4. In die lichte Weite des Transportschlauches 7 bzw. die der Bestrahlungsvorrichtung 9 bzw. die der Kanäle 26, 32 (Fig. 7 und 8) bzw. den Querschnitt des dazwischenliegenden Bereiches können z. B. federbelastete Schieber 30 od. dgl. verstellt werden, gegen die nach dem Einschieben der Umhüllung 20 in die Bestrahlungsvorrichtung 9 bzw. die Kanäle 26, 32 das Ende 20' der Umhüllung 20 in Anlage kommt. Die Schieber 30 engen den Querschnitt jedoch nur so weit ein, daß die Umhüllung 20 an einer Weiterbewegung aus der Bestrahlungsvorrichtung 9 bzw. den Kanälen 26, 32 heraus gehindert wird, während der Klemmkörper 21 bzw. der Magnetkörper 22 ungehindert passieren können. Wenn man eine eingeführte Umhüllung 20 herausziehen will, so muß man die Schieber 30 z. B. händisch in eine Stellung bringen, in der sie die lichte Weite im Bereich zwischen dem Transportschlauch 7 und der Bestrahlungsvorrichtung 9 bzw. den Kanälen 26, 32 nicht beschränken und die Umhüllung 20 kann sodann herausgezogen werden. Eine derartige Auseinanderbewegung der Schieber 30 kann z. B. dadurch erfolgen, daß der Transportschlauch 7 gegen abgeschrägte Endflächen 30' der Schieber 30 angedrückt wird, so daß die Schieber 30 nach außen gedrückt werden.

Beim Einführen der Umhüllung 20 können die gegebenenfalls abgeschrägten Schieber 30 auch durch das spitze bzw. sich verjüngende Ende der Umhüllung 20, z. B. gegen Federkraft, auseinanderbewegt werden.

Fig. 5b zeigt das Passieren der Umhüllung 20 durch die Schieber 30.

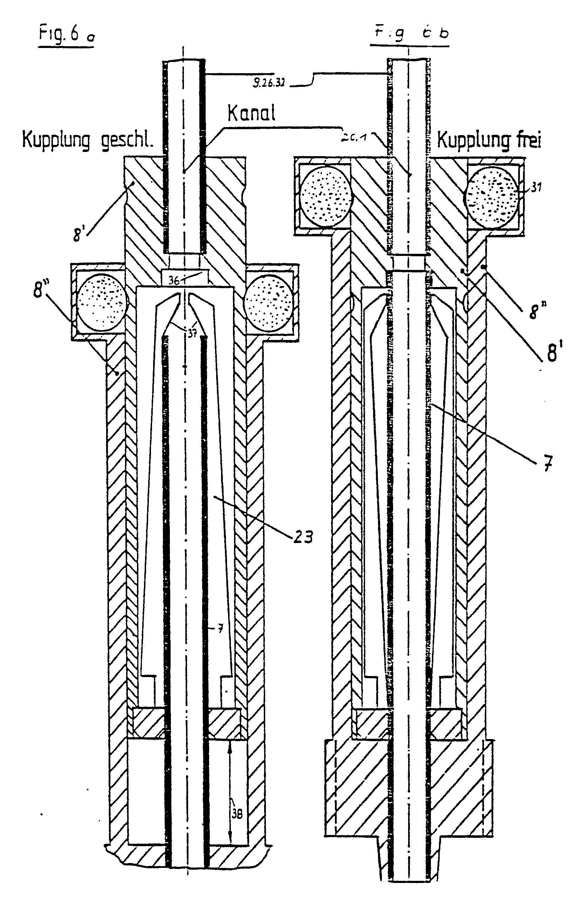

Fig. 6a und 6b zeigen ein Beispiel für eine erfindungsgemäße Kupplungsvorrichtung 8 für eine Bestrahlungsvorrichtung 9 oder Kanäle 26 bzw. 32 gemäß Fig. 7 und 8. In einen Aufnahmeteil 8' ist die Bestrahlungsvorrichtung 9 einführbar bzw. es ist dieser Aufnahmeteil 8' in den Kanal 26 bzw. 32 fortgesetzt. Eine Hülse 8" ist am Aufnahmeteil 8' zwischen einer Durchlaßstellung (Fig. 6b) und einer Hemmstellung (Fig. 6a) verschiebbar gelagert (Pfeil 38). Der Transportschlauch 7 ist durch das Ende des Aufnahmeteiles 8' und das Ende der Hülse 8" geführt, so daß sein Ende dem Ende der Bestrahlungsvorrichtung 9 bzw. einem Kanal 26 bzw. 32 gegenüberliegt. Der Aufnahmeteil 8' besitzt in seinem Endbereich Federn 23, deren freie Enden hakenförmig nach innen ragen und in der Stellung gemäß Fig. 6a in den Raum zwischen den Enden des Transportschlauches 7 und der Bestrahlungsvorrichtung 9 bzw. den Kanälen 26, 32 ragen.

Die Innenseiten der Enden der Federn 23 besitzen Abschrägungen 37, gegen die das Ende des Transportschlauches 7 oder das Ende eines diesen ersetzenden Rohres beim Zusammenschieben der Kupplung bzw. von Hülse 8" und Aufnahmeteil 8' andrückbar ist. Dadurch werden die Federn 23 gespreizt und ein Durchlaß zum ungehinderten Einführen oder zum Herausheben der Strahlenquelle 1 geschaffen. Ein Einführen ist prinzipiell auch dann möglich, wenn die Federn durch die vorne verjüngte Umhüllung 20 auseinandergedrückt werden.

In der Stellung gemäß Fig. 6b liegt das Ende des Transportschlauches 7 gegen einen Anschlag 36 an und gegenüber dem Ende der Bestrahlungsvorrichtung 9 bzw. den Kanälen 26 bzw. 32.

Um die Federn 23 in die Hemmstellung rückzuführen genügt es, den Transportschlauch 7 durch Entfernen der Teile 8' und 8" zurückzuziehen, so daß die Federenden nur einen Durchlaß für das Transportseil 4 bzw. die Teile 21 bzw. 22 freilassen.

Der Transportschlauch 7 ist in der Hülse 8" befestigt und gleitet durch das Ende des Aufnahmeteiles 8'.

Die Federn 23 sind gegebenenfalls in die Hemmstellung vorgespannt und werden im Zuge der Verschiebung der Hülse 8" in die Stellung gemäß Fig. 6b federnd verstellt.

Der Transportschlauch 7 kann im inneren der Hülse 8" bzw. im Aufnahmeteil 8' von einem eigenständigen Teil, z. B. einem Rohr, gebildet sein, und der Transportschlauch 7 kann mit dem Ende der Hülse 8" bzw. dem Rohr in irgendeiner Weise verbunden sein.

Rasten 31 legen die gegenseitigen Endstellungen von Hülse 8" und Aufnahmeteil 8' fest.

Fig. 7 zeigt einen Lagerbehälter 24 mit einer Vielzahl von Lagerkanälen 32 zur Aufbewahrung von Strahlenquellen 1. Es ist nun einfach möglich, mit der erfindungsgemäßen Kupplungsvorrichtung die in Umhüllungen 20 abgelegten Strahlenquellen 1 aus dem Lagerbehälter 24 herauszuholen bzw. dort abzulegen. Dazu ist an jedem Kanal 32 ein Kupplungsteil 8' angeordnet, auf den die entsprechende Hülse 8" am Ende des Transportschlauches 7 aufsteckbar ist, so daß ohne mit den Strahlenquellen 1 in näheren Kontakt zu kommen, eine Entnahme möglich ist.

Um die entnommene Strahlenquelle 1 hinsichtlich Länge, Aktivität und Lage in der Umhüllung 20 überprüfen zu können, ist im Lagerbehälter 24 eine Meßanordnung 25 mit einem Meßkanal 26 mit Meßsonden 34 eingebaut, dessen Eingangsende einen Aufnahmeteil 8' der Kupplung aufweist.

Fig. 8 zeigt eine mobile Meßanordnung 27 mit einem Meßkanal 26 mit Strahlungsdetektoren 34, dessen eines Ende mit einem Aufnahmeteil 8' und dessen anderes Ende mit einer Hülse 8" versehen ist, so daß diese Meßeinrichtung 27 mit dem einen Ende auf einen Aufnahmeteil 8' eines Lagerkanals 32 gesteckt werden kann und an das andere Ende der Meßeinrichtung 27 die Hülse 8" eines Transportschlauches 7 angeschlossen werden kann. Auf diese Weise können, z. B. sofern der Lagerbehälter keine Meßeinrichtung 25 besitzt, im Durchbewegen die Strahlenquellen untersucht werden.

**Patentansprüche**

1. Medizinisch-therapeutische Vorrichtung zum gefahrlosen Einbringen von radioaktiven Strahlenquellen (1) in in den menschlichen Körper eingebettete Bestrahlungsvorrichtungen (9), vorzugsweise in interstitiell eingebrachte, hohle Bestrahlungsnadeln, insbesondere zur low-dose-rate-Bestrahlung, wobei die Vorrichtung aus einem oder mehreren identisch aufgebauten Modulen, bestehend aus je einer strahlenschutzmäßig abgeschirmten Belade- und bzw. oder Lagerstation für die jeweilige Strahlenquelle (1), je einer Beförderungseinrichtung mit einer in einer Hülle (Transportschlauch) (7) geführten flexiblen Schubstange (Transportseil) (4) und je einer von der Beförderungseinrichtung abkoppelbaren Bestrahlungsvorrichtung (9), dadurch gekennzeichnet, daß keine Verbindung zwischen der Strahlenquelle (1) und der flexiblen Schubstange der Beförderungseinrichtung vorgesehen ist, so daß die Strahlenquelle in der Bestrahlungsposition verbleibt, wenn nach Erreichen der Bestrahlungsposition die flexible Schubstange in die Ausgangslage zurückgeführt wird, oder daß zwischen der Strahlenquelle (1) und der flexiblen Schubstange der Beförderungseinrichtung eine in der Bestrahlungsposition die Verbindung zwischen Schubstange und Strahlenquelle freigebende Kupplung (20, 21, 22, 22') vorgesehen ist.

2. Vorrichtung nach Anspruch 1, dadurch gekennzeichnet, daß die Belade- und Lagerstation aus einen gekrümten Rohr (2) besteht, welches durch mechanische Belastung durch die Beförderungseinrichtung zusätzlich verbiegbar ist und daß gegebenenfalls das Rohr (2) in einer, entsprechend den Strahlenschutzerfordernissen dimensionierten Strahlenabschirmung (3), vorteilhafterweise aus Schwermetall, insbesondere aus Blei und/oder Wolfram und/oder abgereichertem Uran, frei beweglich montiert ist.

3. Vorrichtung nach Anspruch 2, dadurch gekennzeichnet, daß die Hülle der Beförderungseinrichtung einen insbesondere durchsichtigen Transportschlauch (7) umfaßt, der über eine Kupplung (6) mit dem Rohr (2) und/oder über eine Kupplung (8) mit der Bestrahlungsvorrichtung (9), insbesondere über jeweils eine Steckverbindung, an- bzw. abkuppelbar ist.

4. Vorrichtung nach einem der Ansprüche bis 3, dadurch gekennzeichnet, daß die flexible Schubstange (4), vorteilhafterweise ein ummanteltes Stahlseil, zur Verschiebung der Strahlenquelle (1) von einem Motor, insbesondere einem Schrittmotor, über eine Antriebsrolle (5) und einer vorteilhafterweise im Anpreßdruck regelbaren Anpreßrolle (15) angetrieben ist.

5. Vorrichtung nach einem der Ansprüche 1 bis 4, dadurch gekennzeichnet, daß zur Überprüfung des Erreichens der Bestrahlungsposition durch die Strahlenquelle (1), insbesondere des Erreichens des Endes der Bestrahlungsvorrichtung (9), eine zusätzliche Krümmung des Rohres (2), welches an einem Ende mittels einer Halterung (11) fixiert ist und am anderen Ende in einer Führung (12) frei beweglich gelagert ist, herangezogen wird und daß zur Feststellung dieser Änderung einer Detektiervorrichtung, insbesondere ein Schalter vorgesehen ist, mit dem der Drehsinn des Motors umschaltbar ist.

6. Vorrichtung nach einem der Ansprüche 1 bis 4, dadurch gekennzeichnet, daß zur Feststellung des Erreichens der Bestrahlungsposition, insbesondere des Endes der Bestrahlungsvorrichtung (9), die Stromaufnahme des Antriebsmotors, insbesondere des Schrittmotors, überwacht ist und daß dessen Änderung bei Erreichen der Bestrahlungsposition bedingt durch den zusätzlichen mechanischen Transportwiderstand durch das Transportseil zum Umschalten des Drehsinns des Antriebsmotors herangezogen ist.

7. Vorrichtung nach einem der Ansprüche 1 bis 6, dadurch gekennzeichnet, daß die Schrittzahl des Schrittmotors durch elektronische Zählung überwacht ist und daß das Umschalten des Drehsinns des Schrittmotors außerhalb eines vorgegebenen Schrittzahlbereiches ein Störsignal auslöst, das zur Abschaltung des Transportes führt.

8. Vorrichtung nach einem der Ansprüche 1 bis 7, dadurch gekennzeichnet, daß zur Überprüfung des Vorhandenseins einer Strahlenquelle (1) im Rohr (2) ein insbesondere in der Abschirmung montierter Strahlendetektor (13), vorteilhafterweise ein strahlungsdosisleistungsabhängiger Widerstand, z. B. eine Photodiode, vorgesehen ist.

9. Vorrichtung nach einem der Ansprüche 1 bis 8, dadurch gekennzeichnet, daß die Strahlenquelle (1) in einer Umhüllung (20), vorzugsweise einem Kunststoffschlauch angeordnet ist, der bei der Einbringung der Strahlenquelle (1) in die Beförderungsvorrichtung das der Beförderungseinrichtung zugewandte Ende der Strahlenquelle (1) überragt.

10. Vorrichtung nach Anspruch 9, dadurch gekennzeichnet, daß das der Beförderungseinrichtung abgewandte Ende der Umhüllung sich zu einer Spitze, z. B. kegelig, verjüngt.

11. Vorrichtung nach Anspruch 9 oder 10, dadurch gekennzeichnet, daß die Umhüllung (20) zumindest im überstehenden Bereich (33) flexibel bzw. elastisch ausgebildet ist und daß am vorderen Ende des Transportseiles (4) ein in das offene Ende der Umhüllung (20) mit Preß- bzw. Reibungssitz einführbarer Klemmkörper (21) vorgesehen ist.

12. Vorrichtung nach Anspruch 9 oder 10, dadurch gekennzeichnet, daß im überstehenden Bereich (33) der Umhüllung (20) von dieser ein magnetischer bzw. magnetisierbarer Körper (22') aufgenommen ist und daß am vorderen Ende des Transportseiles (4) ein Magnet befestigt ist.

13. Vorrichtung nach einem der Ansprüche 1 bis 12, dadurch gekennzeichnet, daß in der Kupplung (8) zwischen dem Ende der Hülle (7) und dem vorzugsweise in die Kupplung (8) einsteckbaren Ende der Bestrahlungsvorrichtung (9) in die lichte Weite des Transportschlauches (7) bzw. der Bestrahlungsvorrichtung (9) verstellbare Trenneinrichtungen, z. B. Schieber, Federn, vorgesehen sind, die in ihrer inneren Endstellung eine Öffnung begrenzen und beim Einfahren mechanisch von Betätigungseinrichtungen oder von der Umhüllung (20) in Durchlaßstellung auseinander bewegbar sind, und welche Öffnung zum ungehinderten Durchlaß des Transportseiles (4) und zum Zurückhalten der Umhüllung (20) in der Bestrahlungsvorrichtung (9) kleiner ist als die der Beförderungseinrichtung zugewandte Querschnittsfläche der Umhüllung (20).

14. Vorrichtung nach Anspruch 13, dadurch gekennzeichnet, daß die Kupplung (8) von einer Hülse (8") und einem Aufnahmeteil (8') gebildet ist, wobei der Transportschlauch (7) mit der Hülse (8") verbunden bzw. verbindbar ist, in der der Transportschlauch bzw. ein diesen verlängerndes Rohrgehalten ist, welche Hülse (8") auf den Aufnahmeteil (8') aufsteckbar ist, der mit der Bestrahlungsvorrichtung (9) oder einem Lager- oder Meßkanal für die Strahlenquellen (1) verbunden bzw. verbindbar ist.

15. Vorrichtung nach Anspruch 14, dadurch gekennzeichnet, daß der Aufnahmeteil (8') Federn aufweist, in die der Transportschlauch (7) bzw. ein diesen verlängerndes Rohr einführbar ist, daß die Federenden eine schräge innere Auflauffläche (37) aufweisen und vom Transportschlauch (7) bzw. dem Rohr oder der Umhüllung (20) der Strahlenquelle (1) spreizbar bzw. auseinanderdrückbar sind.

16. Vorrichtung nach Anspruch 14 oder 15, dadurch gekennzeichnet, daß Rastvorrichtungen (31) zwischen der Hülse (8") und dem Aufnahmeteil (8') vorgesehen sind.

17. Vorrichtung nach einem der Ansprüche 14 bis 16, dadurch gekennzeichnet, daß der Aufnahmeteil (8') einen Anschlag (36) für den Transportschlauch (7) bzw. das Rohr besitzt.

18. Vorrichtung nach einem der Ansprüche 1 bis 17, dadurch gekennzeichnet, daß die Belade-und Lagerstation einen Lagerbehälter (24) umfaßt, in dem Strahlenquellen (1) einzeln in Kanälen (32) gelagert sind, von denen jeder an seinem Ende einen mit der Hülse (8") am Ende des Transportschlauches (7) verbindbaren bzw. in diesen einführbaren Trenneinrichtungen aufweisenden Anschlußteil (8') aufweist.

19. Vorrichtung nach Anspruch 18, dadurch gekennzeichnet, daß im Lagerbehälter (24) ein stationärer Meßkanal (25) zur Aktivitäts- und/oder Längenmessung der Strahlenquellen (1) vorgesehen ist, dessen Aufnahmeöffnung einen mit der Hülse (8") verbindbaren Trenneinrichtungen aufweisenden Anschlußteil (8') aufweist.

20. Vorrichtung nach einem der Ansprüche 1 bis 19, dadurch gekennzeichnet, daß ein einen Meßkanal (26) aufweisender mobiler Strahlendetektor (27) mit Strahlendetektoren zur Aktivitäts- und/oder Längenmessung der Strahlenquellen (1) vorgesehen ist, der auf einer Seite mit einer Hülse (8") und auf der anderen Seite mit einem Trenneinrichtungen aufweisenden Aufnahmeteil (8') versehen ist.

## Claims

1. Medico-therapeutic apparatus for the safe introduction of radioactive sources (1) into irradiation devices (9) embedded in the human body, preferably into interstitially inserted, hollow irradiation needles, in particular for low dose rate irradiation, the apparatus being composed of one or a plurality of identically formed modules, consisting of one each radioprotectively shielded loading and/or storage station for the respective radioactive source (1), one each transport means having a flexible drive rod (transport cable) (4) guided in a sheath (transport tube) (7) and one each irradiation device (9) suitable for being uncoupled from the transport means, characterized in that no connection is provided between the radioactive source (1) and the flexible drive rod of the transport means, so that the radioactive source remains in irradiation position when the flexible drive rod is returned to its starting position after reaching the irradiation position, or that a coupling (20, 21, 22, 22') releasing the connection between drive rod and radioactive source in irradiation position is provided between the radioactive source (1) and the flexible drive rod of the transport means.

2. Apparatus according to claim 1, wherein the loading and storage station consists of a curved tube (2) which is additionally bendable by mechanical load through the transport means and the tube (2) is optionally freely movably supported in a radiation protection shielding (3) dimensioned according to radiation protection requirements and conveniently consisting of heavy metal, in particular of lead and/or tungsten and/or depleted uranium.

3. Apparatus according to claim 2, wherein the sheath of the transport means comprises an in particular transparent transport tube (7) suitable for being coupled or uncoupled via a coupling (6) with or from the tube (2) and/or via a coupling (8) with or from the irradiation device (9), in particular via one each plugging connection.

4. Apparatus according to any one of the claims 1 to 3, wherein the flexible drive rod (4) is conveniently a sheathed steel cable driven for displacing the radioactive source (1) by a motor, in particular a stepping motor, via a driving roll (5) and a pressure roll (15) of conveniently adjustable compression force.

5. Apparatus according to any one of the claims 1 to 4, wherein the reaching of the irradiation position by the radioactive source (1), in particular the reaching of the end of the irradiation device (9), is checked by means of an additional curvature of the tube (2) which is fixed on one end by means of a holding device (11) and freely movably supported on the other end in a guide means (12) and wherein a detecting means, in particular a switch, by means of which the rotating direction of the motor is reversible, is provided for determining this change.

6. Apparatus according to any one of the claims 1 to 4, wherein the power consumption of the drive motor, in particular the stepping motor, is monitored for determining the reaching of the irradiation position, in particular the end of the irradiation device (9), and its change on reaching the irradiation position is effected by the reversing of the rotating direction of the drive motor caused by the additional mechanical transport resistance by the transport cable.

7. Apparatus according to any one of the claims 1 to 6, wherein the number of steps of the stepping motor is monitored by electronic counting and the reversal of the rotating direction of the stepping motor outside of a predetermined range of number of steps generates a trouble signal which causes the interruption of the transport operation.

8. Apparatus according to any one of the claims 1 to 7, wherein a radiation detector (13), conveniently a resistor subject to the radiation dose rate, for instance a photodiode, in particular mounted in the shielding, is provided for checking the presence of a radioactive source in the tube (2).

9. Apparatus according to any one of the claims 1 to 8, wherein the radioactive source (1) is disposed in a sheathing (20), preferably in a tube of plastics material, which projects beyond the end of the radioactive source (1) facing the transport means on introduction of the radioactive source (1) into the transport means.

10. Apparatus according to claim 9, therein the end of the sheathing facing away from the transport means tapers, for instance conically, to form a point.

11. Apparatus according to claim 9 or 10, wherein the sheathing (20) is formed flexibly or elastically at least in the projecting range (33) and a clamping body (21) insertable into the open end of the sheathing (20) with press fit or friction fit is provided on the front end of the transport cable (4).

12. Apparatus according to claim 9 or 10, wherein a magnetic or magnetizable body (22') is received by the sheathing in its projecting range (33) and a magnet is attached to the front end of the transport cable (4).

13. Apparatus according to any one of the claims 1 to 12, wherein separating means adjustable in the inner diameter of the transport tube (7) or the irradiation device (9), for instance sliding valves or springs, defining an opening in their internal end positions and mechanically movable away from one another on introduction by actuating means or by the sheathing (20) in passing position, which opening for the unimpeded passing of the transport cable (4) and for retaining the sheathing (20) in the irradiation device (9) is smaller than the cross section area of the sheathing (20) facing the transport means, are provided in the coupling (8) between the end of the sheath (7) and the end of the irradiation device (9) preferably insertable into the coupling (8).

14. Apparatus according to claim 13, wherein the coupling (8) is formed by a sleeve (8") and a receiving part (8'), the transport tube (7) being connected or connectable to the sleeve (8") in which the transport tube or a tube extending the transport tube is being supported, which sleeve (8") is pluggable onto the receiving part (8') which is connected or connectable to the irradiation device (9) or a storage or measuring channel for the radioactive sources (1).

15. Apparatus according to claim 14, wherein the receiving part (8') is provided with springs into which the transport tube (7) or a tube extending it is insertable, the ends of the springs have an inclined inner leading surface (37) and are suitable for being spread or forced apart from one another by the transport tube (7) or the tube extending it or the sheathing (20) of the radioactive source (1).

16. Apparatus according to claim 14 or 15, wherein locking means (31) are provided between the sleeve (8") and the receiving part (8').

17. Apparatus according to any one of the claims 14 to 16, wherein the receiving part (8') is provided with a stop (36) for the transport tube (7) or the tube extending it.

18. Apparatus according to any one of the claims 1 to 17, wherein the loading and storage station comprises a storage container (24) in which radioactive sources (1) are stored individually in channels (32) of which each is provided on its end with a connecting part (8') connectable to the sleeve (8") at the end of the transport tube (7) and provided with separating means insertable into the tube (7).

19. Apparatus according to claim 18, wherein a stationary measuring channel (25) for measuring the activity and/or length of the radioactive sources (1) whose inlet opening is provided with a connecting part (8') connectable to the sleeve (8") and provided with separating means is provided in the storage container (24).

20. Apparatus according to any one of the claims 1 to 19, wherein a mobile radiation detector (27) with radiation detectors for measuring the activity and/or length of the radiation sources and having a measuring channel (26) is provided, which radiation detector (27) is provided on one side with a sleeve (8") and on the other side with a receiving part (8') having separating means.

**Revendications**

1. Dispositif médico-thérapeutique pour placer sans danger des sources radioactives (1) dans des dispositifs d'irradiation (9) implantés dans le corps humain, de préférence dans des aiguilles d'irradiation creuses introduites dans les tissus, en particulier pour l'irradiation à faible dose, ce dispositif consistant en un ou plusieurs modules de construction identique, comprenant chacun une station de chargement et/ou de déchargement de la source radioactive correspondante (1), blindée conformément aux exigences de la radioprotection, un dispositif de transport avec une tige coulissante flexible (câble de transport) (4) passant dans une gaine (tuyau de transport) (7) et un dispositif d'irradiation (9) détachable du dispositif de transport, caractérisé en ce qu'il n'est pas prévu de liaison entre la source radioactive (1) et la tige coulissante flexible du dispositif de transport, en sorte que la source radioactive reste en position d'irradiation lorsque la tige coulissante flexible revient à sa position initiale une fois que la position d'irradiation est atteinte, ou en ce qu'il a été prévu entre la source radioactive (1) et la tige coulissante flexible du dispositif de transport un accouplement (20, 21, 22, 22') désolidarisant la tige coulissante et la source radioactive en position d'irradiation.

2. Dispositif selon la revendication 1, caractérisé en ce que la station de chargement et de stockage consiste en un tube coudé (2), qui peut être en outre infléchi sous l'effet d'une contrainte mécanique exercée par le dispositif de transport et en ce que, le cas échéant, le tuyau (2) est monté à déplacement libre dans un blindage (3) dont les dimensions répondent aux exigences de la radioprotection, ledit blindage étant en plomb et/ou en tungstène et/ou en uranium appauvri.

3. Dispositif selon la revendication 2, caractérisé en ce que la gaine du dispositif de transport enferme un tuyau de transport (7), particulièrement un tuyau transparent, qui peut être réuni au ou séparé du tube (2) par un accouplement (6) et/ou réuni au ou séparé du dispositif d'irradiation (9) par un accouplement (8), le couplage pouvant en particulier se faire dans l'un et l'autre cas par emboîtement.

4. Dispositif selon une des revendications 1 à 3, caractérisé en ce que, pour déplacer la source radioactive (1), la tige coulissante flexible (4), qui est avantageusement un câble d'acier sous gaine, est entraînée par un moteur, en particulier un moteur pas-à-pas, par l'intermédiaire d'un galet de commande (5) et d'un galet de pression (15) dont on peut avantageusement régler la force de pression.

5. Dispositif selon une des revendications 1 à 4, caractérisé en ce qu'une inflexion supplémentaire du tuyau (2) dont une extrémité est fixée à un support (11) et dont l'autre extrémité se déplace librement dans un dispositif de guidage (12) permet de vérifier si la source radioactive (1) a atteint la position d'irradiation, et en particulier si elle a atteint l'extrémité du dispositif d'irradiation (9), et en ce qu'il a été prévu un dispositif de détection pour vérifier cette modification, en particulier un commutateur permettant d'inverser le sens de rotation du moteur.

6. Dispositif selon une des revendications 1 à 4, caractérisé en ce que la consommation de courant du moteur, en particulier du moteur pas-à-pas, est surveillée pour constater si la position d'irradiation est atteinte, en particulier à l'extrémité du dispositif d'irradiation (9), la modification de la consommation qui intervient lorsque la position d'irradiation est atteinte, du fait de la résistance mécanique au transport supplémentaire venant du câble de transport, servant à inverser le sens de marche du moteur.

7. Dispositif selon une des revendications 1 à 6, caractérisé en ce que le nombre de pas du moteur pas-à-pas est surveillé par comptage électronique et que l'inversion du sens de rotation du moteur pas-à-pas en dehors d'une plage de pas donnée déclenche un signal de dérangement amenant à couper le transport.

8. Dispositif selon une des revendications 1 à 7, caractérisé en ce que, pour vérifier la présence d'une source radioactive (1) dans le tube (2), il est prévu un détecteur de rayonnement (13) monté en particulier dans le blindage, avantageusement une résistance dépendante de la dose émise, p. ex. une photodiode.

9. Dispositif selon une revendication de 1 à 8, caractérisé en ce que la source radioactive (1) est disposée dans une enveloppe (20), avantageusement dans un tuyau de plastique qui, lorsque la source radioactive (1) est introduite dans le dispositif de transport (4, 7), dépasse de l'extrémité de la source radioactive (1) du côté du dispositif de transport.

10. Dispositif selon la revendication 9, caractérisé en ce que l'extrémité de l'enveloppe opposée au dispositif de transport se termine en pointe, p. ex. en forme de cône.

11. Dispositif selon la revendication 9 ou 10, caractérisé en ce que l'enveloppe (20) est souple ou élastique, tout au moins dans la partie (33) qui dépasse la source radioactive, et en ce qu'il est prévu à l'extrémité avant du câble de transport (4) un corps de blocage (21) pouvant être introduit dans l'extrémité ouverte de l'enveloppe (20) avec ajustement pressé ou à friction.

12. Dispositif selon la revendication 9 ou 10, caractérisé en ce qu'un corps magnétique ou magnétisable (22') se trouve dans la partie (33) de l'enveloppe (20) dépassant la source radioactive et qu'un aimant est fixé à l'extrémité avant du câble de transport (4).

13. Dispositif selon une des revendications 1 à 12 caractérisé en ce que, dans l'accouplement (8), des dispositifs de séparation réglables, p. ex. coulisses, ressorts, sont prévus dans la lumière du tuyau de transport (7) ou du dispositif de transport (9), entre l'extrémité de la gaine (7) et l'extrémité du dispositif d'irradiation (9) s'introduisant avantageusement dans l'accouplement (8), tels qu'en position extrême intérieure ils limitent une ouverture et que, lorsqu'on introduit la source radioactive, ils puissent être écartés mécaniquement par des dispositifs de commande ou par l'enveloppe (20) pour ouvrir le passage, cette ouverture qui permet le passage sans entrave du câble de transport (4) et le maintien de l'enveloppe (20) dans le dispositif d'irradiation (9) étant plus petite que la section de l'enveloppe (20) se trouvant du côté du dispositif de transport.

14. Dispositif selon la revendication 13, caractérisé en ce que l'accouplement (8) consiste en une gaine (8″) et une pièce d'attache (8'), le tuyau de transport (7) étant relié ou pouvant être relié à la gaine (8″) dans laquelle est maintenu le tuyau de transport ou un tube qui le prolonge, ladite gaine (8″) pouvant être emmanchée sur la pièce d'attache (8') reliée ou pouvant être reliée au dispositif d'irradiation (9) ou à un canal de stockage ou de mesure de la source radioactive (1).

15. Dispositif selon la revendication 14, caractérisé en ce que la pièce d'attache (8') comporte des ressorts dans lesquels on peut introduire le tuyau de transport (7) ou un tube qui le prolonge, les extrémités des ressort présentant une surface de butée interne (37) oblique et pouvant être écartées ou ouvertes par le tuyau de transport (7) ou le tube ou l'enveloppe (20) de la source radioactive (1).

16. Dispositif selon la revendication 14 ou 15 caractérisé en ce que des dispositifs de crantage (31) sont prévus entre la gaine (8″) et la pièce d'attache (8').

17. Dispositif selon une des revendications 14 à 16 caractérisé en ce que la pièce d'attache (8') possède une butée (36) pour le tuyau de transport (7) ou le tube.

18. Dispositif selon une des revendications 1 à 17, caractérisé en ce que la station de chargement et de stockage renferme un récipient de stockage (24) dans lequel des sources radioactives (1) sont stockées séparément dans des canaux (32), chacun de ces canaux présentant un raccord (8') muni de dispositifs de séparation, qui peut être relié à la gaine (8″) située à l'extrémité du tuyau de transport (7) ou introduit dans celle-ci.

19. Dispositif selon la revendication 18, caractérisé en ce qu'un canal de mesure stationnaire (25) pour la mesure de l'activité ou de la longueur des sources radioactives (1) est prévu dans le récipient de stockage (24), dont l'orifice d'attache comporte un raccord (8') présentant des dispositifs de séparation, pouvant être relié à la gaine (8″).

20. Dispositif selon une des revendications 1 à 19, caractérisé en ce qu'il est prévu un détecteur mobile de rayonnement (27) présentant un canal de mesure (26), avec des détecteurs radiosensibles pour la mesure de l'activité et/ou de la longueur des sources radioactives (1), muni d'un côté d'une gaine (8″), de l'autre d'une pièce d'attache (8') comportant des dispositifs de séparation.

Fig. 1

Fig. 2

# Fig. 3 Funktionsdiagramm

```
        ┌─────────┐                                    ┌─────────┐
        │  START  │                                    │  ENDE   │
        └────┬────┘                                    └────┬────┘
             │          ┌──────────────────┐          nein │
             │          │  Fehlermeldung   │               │
             ▼          └────────▲─────────┘            ◇ Endkontakt ──ja──► ┌──────────────────┐
         ◇ Schlauch ──nein───────┘                     Störung            │ Fehler: alle Lamp-│
         angeschl.?                                        !               │ chen blinken      │
             │                                             ▲               └──────────────────┘
             ja                                            │
             ▼                                        ┌──────────┐
         ◇ Strahler ok! ──nein──────────────────────►│ Rückholen│
             │                                        └─────▲────┘
             ja                                             │
             ▼                                              │
         ◇ End-kontakt im ──nein───┘                        │
           Bereich?                                         │
             │                                              │
             ja ─────────────────────────────────────────┘
```

Fig. 4 a

Fig. 4 b

4

22

21

7

22'

33

20

1

Fig. 5 a

Fig. 5 b

21

7

30'

30

20'

Federkraft

1

20

9

5

Fig. 6 a

Fig. 6 b

Kanal

9.26.32

Kupplung geschl.

Kupplung frei

8'

8''

36

37

31

8''

8'

7

23

7

38

## Fig 7

## Fig. 8